Europäisches Patentamt

European Patent Office

Office européen des brevets

(11) Numéro de publication : **0 186 551**
**B1**

(12) **FASCICULE DE BREVET EUROPÉEN**

(45) Date de publication du fascicule du brevet :
24.01.90

(51) Int. Cl.⁵ : **A 61 K 39/395**

(21) Numéro de dépôt : **85402319.9**

(22) Date de dépôt : **27.11.85**

(54) **Médicament comportant en tant qu'association au moins une immunotoxine et au moins un polymère contenant du mannose.**

(30) Priorité : **29.11.84 FR 8418203**

(43) Date de publication de la demande :
**02.07.86 Bulletin 86/27**

(45) Mention de la délivrance du brevet :
**24.01.90 Bulletin 90/04**

(84) Etats contractants désignés :
**AT BE CH DE FR GB IT LI LU NL SE**

(56) Documents cités :
**EP--A-- 0 080 401**
**EP--A-- 0 086 152**
**EP--A-- 0 088 694**
**EP--A-- 0 089 270**
**EP--A-- 0 089 880**

(73) Titulaire : **SANOFI S.A.**
**40, Avenue George V**
**F-75008 Paris (FR)**

(72) Inventeur : **Casellas, Pierre**
**24 bis, rue Lakanal**
**F-34100 - Montpellier (FR)**
Inventeur : **Bourrie, Bernard Rés. du Plan des 4**
**Seigneurs**
**300, rue des Brusses 2 - Bâtiment B**
**F-34100 - Montpellier (FR)**
Inventeur : **Gros, Pierre**
**18, rue des Muriers**
**F-34100 - Montpellier (FR)**

(74) Mandataire : **Gillard, Marie-Louise et al**
**Cabinet Beau de Loménie 55, Rue d'Amsterdam**
**F-75008 Paris (FR)**

Dans les brevets français antérieurs n° 78/27838 et addition n° 79/24665 et dans les demandes de brevets français n° 81/07596 et n° 81/21836, la demanderesse a décrit la préparation de produits anticancéreux dits conjugués, obtenus par couplage, par liaison covalente, de la chaîne A de ricine avec des anticorps ou fragments d'anticorps dirigés contre un antigène porté par la cellule à détruire. Les produits de ce type sont désignés dans la présente demande sous le nom générique d'Immunotoxines.

Dans ses demandes de brevets français n° 81/21836, n° 82/02091, n° 82/04119, n° 82/04047 et n° 82/04547, la demanderesse a également montré la capacité de certaines substances (sels d'ammonium, ionophores carboxyliques monovalents, méthylamine, chloroquine et conjugués enzymatiques susceptibles de libérer de l'ammoniac) de potentialiser l'action cytotoxique des immunotoxines.

Toutefois, les effets thérapeutiques des immunotoxines activées ou non ne peuvent se manifester pleinement que dans la mesure où l'immunotoxine peut, par sa partie anticorps, se localiser in vivo, sous forme active, sur la cible cellulaire à détruire (condition sine qua non à toute expression d'activité des immunotoxines). La capacité de l'immunotoxine à se localiser sur la cible dépend en tout premier lieu de l'aptitude de l'immunotoxine à demeurer dans la circulation sanguine et les fluides extra-cellulaires sous forme active pendant des temps suffisants pour qu'elle atteigne sa cible cellulaire et à des concentrations suffisamment fortes pour que le taux d'occupation des sites antigéniques correspondants soit élevé.

La demanderesse a effectué de nombreuses études qui ont permis d'établir la cinétique d'élimination plasmatique des immunotoxines après injection intraveineuse dans différents modèles animaux. Il est apparu que, après l'injection, le taux plasmatique d'immunotoxine biologiquement active décroît très rapidement et de façon très importante. Ainsi, typiquement, chez le lapin, dans un modèle utilisant une immunotoxine construite en couplant à l'aide d'un bras à pont disulfure la chaîne A de ricine à un anticorps monoclonal dirigé contre l'antigène T65 des lymphocytes T humains, il apparaît que 97 % de l'immunotoxine présente dans le sang circulant au temps 0 de l'injection disparaissent en 30 min et 99,9 % en 17 h. Les résultats obtenus sont analogues si le bras reliant l'anticorps à la chaîne A de ricine comporte une liaison thioéther au lieu d'une liaison disulfure. Cette rapide disparition de l'immunotoxine est bien évidemment préjudiciable à l'expression de sa capacité cytotoxique complète, en empêchant que l'immunotoxine ne sature de façon durable une proportion élevée des antigènes cibles portés par les cellules à détruire. En outre, la comparaison des cinétiques d'élimination plasmatique des immunotoxines avec celles des anticorps non conjugués correspondants montre, à l'inverse, que les anticorps — comme cela est bien connu — se maintiennent dans le plasma à un haut niveau pendant des temps relativement longs. Or, il existe toujours, même dans les préparations d'immunotoxines les plus purifiées, un certain taux résiduel d'anticorps non conjugués. Par le jeu des vitesses différentielles d'élimination des immunotoxines et des anticorps, progressivement les anticorps non conjugués, initialement très minoritaires, deviennent majoritaires au bout de quelques heures et donc, progressivement, ces anticorps deviennent par compétition de puissants antagonistes pour la fixation des immunotoxines sur leurs cibles.

De ces études, apparaît clairement l'intérêt d'accroître la persistance plasmatique des immunotoxines, sous forme active, afin d'augmenter à la fois la durée et le taux d'occupation des antigènes-cibles et, par conséquent, d'améliorer les effets thérapeutiques des immunotoxines.

La présente invention a pour objet des nouveaux médicaments permettant d'inhiber l'élimination rapide des immunotoxines du plasma après injection, sans qu'il soit porté atteinte aux propriétés intrinsèques caractéristiques des immunotoxines.

Les médicaments selon l'invention, conditionnés de façon à être utilisés par voie injectable, de préférence par voie intraveineuse, comportent, en association, au moins une immunotoxine obtenue à partir de toxine ou sous-unité toxique ou fragment de sous-unité toxique naturelle ou semi-synthétique ou synthétique comportant des groupements polysaccharidiques contenant des résidus de mannose, en particulier en position terminale, quel que soit l'anticorps constitutif et quel que soit le type de liaison choisi pour relier l'anticorps à la toxine ou sous-unité toxique ou fragment toxique, et au moins un polymère glucidique, polyosidique ou polysaccharidique de poids moléculaie moyen supérieur à 1 000 comportant de 20 à 100 % de résidus mannose, quel que soit le type d'enchaînement reliant ces résidus mannose entre eux ou à d'autres sucres.

D'une façon surprenante, il a été trouvé, selon la présente invention, que les mannanes représentent un type de substances particulièrement intéressantes pour accroître les taux plasmatiques des immunotoxines. Le terme de mannane est employé ici pour désigner tout polymère glucidique, polyoside ou polysaccharide, de poids moléculaire moyen supérieur à 1 000 comportant une proportion importante de résidus mannose et plus particulièrement de 20 à 100 % de résidus mannose, quel que soit le type d'enchaînement osidique reliant ces résidus mannose entre eux ou à d'autres sucres. En particulier et, à titre d'exemple non limitatif, on peut utiliser au sens de la présente invention des mannanes naturels isolés à partir de levures (par exemple Saccharomyces cerevisiae). Il s'agit alors de la fraction glucidique d'un peptidoglycane appartenant à la paroi cellulaire de ces levures. Le complexe protéine-mannane est un mélange de macromolécules dans lequel le composant polysaccharidique représente 50 à 90 % du complexe. La fraction mannane est elle-même un polymère de D-mannose. Elle est formée d'une armature de résidus mannose couplés en position $\alpha 1 \rightarrow 6$ à laquelle s'ajoutent des chaînes latérales de différentes longueurs comportant des liaisons $\alpha 1 \rightarrow 3$ et $\alpha 1 \rightarrow 2$.

D'une façon surprenante, le mannane, utilisé à des doses où il ne présente ni en lui-même, ni en association avec l'immunotoxine aucune toxicité pour l'animal, permet d'accroître de façon extrêmement importante (d'un facteur de l'ordre de 100) et durable la concentration plasmatique des immunotoxines, ce qui améliore considérablement leur localisation sur la cible et évite des phénomènes d'inhibition de fixation dus à la présence d'anticorps libres dans les préparations, comme cela est indiqué précédemment.

L'absence remarquable de toxicité des mannanes en fait des substances de choix pour une utilisation pharmaceutique en association avec les immunotoxines. L'association de l'immunotoxine au mannane n'accroît pas de façon importante la toxicité propre de l'immunotoxine. Cette association n'interfère pas non plus avec les propriétés de cytotoxicité spécifique caractéristiques des immunotoxines en présence ou non des potentialisateurs déjà décrits.

Par ailleurs, des expériences de localisation in vivo de l'immunotoxine radiomarquée injectée chez des animaux sans cible spécifique ont montré que, dans les premières minutes après l'injection, le conjugué se localise préférentiellement dans le foie. Il en est de même pour la chaîne A qui suit le même devenir lorsqu'elle est injectée sous forme non couplée. Cela suggère fortement que l'immunotoxine se fixe dans le foie par l'intermédiaire de la chaîne A de ricine qu'elle contient. Il est connu que la chaîne A de ricine est une glycoprotéine donc les groupements polyosidiques comprennent des résidus de mannose et de N-acétylglucosamine, les résidus de mannose étant en position terminale (Agri. Biol. Chem. (1978) 42, 501). Il a été également établi l'existence au niveau du foie de récepteurs capables de reconnaître des glycoprotéines ayant de tels résidus de mannose terminaux.

Il a été ainsi montré que les glycoprotéines reconnues par ces récepteurs — présents essentiellement sur les cellules de Kupffer — sont rapidement éliminées de la circulation sanguine par fixation sur ces cellules qui les métabolisent. Ceci est bien documenté notamment dans le cas de la $\beta$-glucuronidase, ainsi que dans le cas de la ribonucléase B (Arch. Biochem. Biophys. (1978) 188, 418 ; Advances in Enzymology Meister A. Ed New York (1974) ; Pediat. Res. (1977) 11, 816).

De l'ensemble de ces données, il apparaît que la rapide élimination des immunotoxines peut s'expliquer par la reconnaissance des résidus mannose de la chaîne A de ricine par les cellules hépatiques et en particulier les cellules de Kupffer. La propriété des mannanes d'inhiber l'élimination plasmatique rapide des immunotoxines à chaîne A de ricine s'explique en outre aisément par le fait que les mannanes administrés occupent les récepteurs cellulaires des glycoprotéines et s'opposent donc à la reconnaissance par ces récepteurs des motifs polyosidiques portés par la chaîne A de ricine ou par tout conjugué la contenant.

La propriété des mannanes d'inhiber l'élimination plasmatique rapide des immunotoxines à chaîne A de ricine s'applique aussi bien, pour les raisons indiquées plus haut, à la chaîne A de ricine non couplée ou à toute molécule hybride, naturelle ou semi-synthétique, contenant la chaîne A de ricine et, en particulier, à toutes les immunotoxines contenant la chaîne A de ricine, quel que soit le type de liaison choisi pour relier l'anticorps à la chaîne A de ricine. Cette propriété des mannanes s'applique plus généralement pour les mêmes raisons à tout immunotoxine, quelle que soit la toxine utilisée pour la produire, si cette toxine ou sous-unité toxique comporte des groupements polysaccharidiques contenant des résidus de mannose, en particulier en position terminale, quel que soit l'anticorps constitutif et quel que soit le type de liaison choisi pour relier l'anticorps à la toxine ou sous-unité toxique.

Les exemples suivants permettent de mieux comprendre l'invention sans en limiter la portée.

## Exemple 1

Cet exemple a pour objet de mettre en évidence les modifications de cinétique d'élimination des immunotoxines (ainsi que de celles de leurs éléments constitutifs) en présence ou en absence de mannane.

A - Les modes opératoires suivants ont été utilisés :

a) Mesure de la cinétique d'élimination de l'immunotoxine IT-T101.

Le conjugué appelé IT-T101 est obtenu par réaction entre un anticorps anticellules T humaines (anticorps T101 dirigé contre l'antigène T65) substitué par un groupe disulfure activé et la chaîne A de ricine. La préparation et les propriétés cytotoxiques de ce conjugué ont été décrites dans la demande de brevet français n° 8121836 de la demanderesse. Le conjugué IT-T101 est administré chez le lapin par injection unique dans une veine de l'oreille. La quantité injectée correspond à 1,25 mg d'immunotoxine par kg de poids corporel, soit 0,415 mg/kg exprimé en chaîne A, et 0,835 mg/kg exprimé en anticorps. Les échantillons de sang sont prélevés au cours du temps sur héparine. Les plasmas sont analysés à l'aide d'un test immunoradiométrique ci-après désigné par l'abréviation IRM-1.

Cette technique a l'avantage de doser l'immunotoxine sans modification de celle-ci. Ce dosage est réalisé dans des plaques à microtitrations (par exemple : « NUNC-TSP screening system » Poly Labo Block, France) dont le couvercle est muni de pointes hyper-absorbantes qui plongent dans les puits du socle. Ces pointes constituent les phases solides. Des anticorps de brebis antichaîne A de ricine (ci-après désignés sous l'abréviation AcI), purifiés par chromatographie d'affinité, sont adsorbés sur les phases solides. Pour cela, 200 μl d'une solution d'Acl à 10 μg/ml dans le tampon phosphate 20 mM pH 7, NaCl 150 mM sont distribués dans les puits. Les pointes sont mises en contact d'abord avec la solution d'Acl pendant 24 h à 4 °C et ensuite avec du sérum de veau fœtal pendant 3 h à 20 °C pour saturer tous les sites de fixation. L'immunoabsorbant saturé est alors mis en contact pendant 3 h à 20 °C avec les échantilons plasmatiques à différentes dilutions ou avec des solutions d'immunotoxine IT-T101 de concentrations connues pour l'établissement de la courbe d'étalonnage. Un lavage est effectué par un tampon phosphate 20 mM pH 7, NaCl 150 mM puis l'immunoabsorbant est mis en contact pendant 2 h à 20 °C avec des anticorps de chèvre anti-IgG de souris purifiés par chromatographie d'affinité et radiomarqués (ci-après désignés par l'abréviation Ac2). Le radiomarquage de l'Ac2 est réalisé avec l'iode 125 en présence de chloramine T selon la méthode de Greenwood et Hunter (Biochem. J., (1963) 89, 114) ; l'activité spécifique des Ac2 radiomarqués est de 5 à 10 μCi/mg. $10^6$ cpm d'Ac2 radiomarqués sont introduits sous 200 ml dans un tampon phosphate 20 mM pH 7, NaCl 150 mM, sérum albumine bovine 0,1 %. Après lavage, dans un tampon phosphate 20 mM pH 7, NaCl 150 mM, les pointes sont détachées et la quantité d'Ac2 liée est mesurée par comptage de la radioactivité. La mesure de la concentration en immunotoxine dans les échantillons à doser se fait par référence à la courbe d'étalonnage réalisée avec l'IT-T101 introduite à différentes concentrations connues.

Ce test — par son principe de reconnaissance — permet de mesurer les molécules d'immunotoxine intactes.

De plus, la comparaison des concentrations obtenues dans cet essai avec celles mesurées selon le test d'activité cytotoxique in vitro sur cellules cibles donne des valeurs identiques, ce qui assure que l'immunotoxine dosée par le test IRM-1 correspond à des molécules qui ont conservé leur propriété de cytotoxicité.

b) Mesure de la cinétique d'élimination de l'anticorps anticellules T humaines (ou anticorps T101).

Cet anticorps a été préparé et purifié ainsi qu'il a été indiqué dans la demande de brevet français n° 8121836. L'anticorps T101 est injecté chez le lapin par voie intraveineuse à la dose de 0,835 mg/kg. Les prélèvements plasmatiques sont effectués comme précédemment. La concentration en anticorps dans les échantillons est mesurée par dosage radioimmunométrique (IRM-2). Cet essai est réalisé dans les mêmes conditions que le test IRM-1, excepté que la solution Acl est ici une solution à 10 mg/ml d'anticorps de chèvre anti-IgG de souris purifiés par chromatographie d'affinité. L'anticorps Ac2 est identique à celui du test IRM-1. La mesure de la concentration en anticorps T101 dans les échantillons à doser se fait par référence à la courbe d'étalonnage réalisée avec l'anticorps T101 introduit à différentes concentrations connues.

c) Mesure de la cinétique d'élimination de la chaîne A de ricine.

La chaîne A de ricine a été préparée et purifiée ainsi qu'il a été indiqué dans les brevets français antérieurs n° 78/27838 et addition n° 79/24655. La chaîne A est injectée chez le lapin par voie intraveineuse à la dose de 0,415 mg/kg. Les prélèvements plasmatiques sont effectués comme précédemment. La concentration en chaîne A dans les échantillons est mesurée par dosage immunoradiométrique (IRM-3). Cet essai est réalisé dans les mêmes conditions que le test IRM-1, les anticorps Acl, absorbés sur la phase solide, étant également des anticorps de brebis antichaîne A de ricine purifiés par chromatographie d'affinité et les anticorps Ac2 étant les mêmes anticorps radiomarqués comme décrits dans IRM-1. La mesure de la concentration en chaîne A de ricine dans les échantillons à doser se fait par référence à une courbe d'étalonnage réalisée avec la chaîne A de ricine introduite à différentes concentrations connues.

Les valeurs des concentrations en immunotoxine, anticorps et chaîne A de ricine dans les plasmas, mesurées par ces trois tests, sont reproductibles, fiables et quantitatives. Le seuil de détection pour ces trois produits est de 1 ng/ml. L'étude de la reproductibilité intra- et inter-essais donne des coefficients de variation inférieurs à 10 % pour les valeurs de concentration comprises dans la gamme de 1 à 200 ng/ml.

B - Résultats :

Les résultats des expériences effectuées sont représentés sous la forme de courbes présentant en abscisses le temps, exprimé en heures, et, en ordonnées, sur une échelle logarithmique, la concentration du produit mesuré, rapportée en pourcent de la concentration plasmatique théorique au temps zéro. Cette valeur appelée « concentration plasmatique relative » (CPR) est calculée à l'aide de l'expression suivante :

$$CPR = \frac{\text{concentration mesurée au temps T}}{\text{quantité injectée/volume plasmatique}} \times 100$$

Le volume plasmatique est considéré égal à 36 ml/kg de poids corporel de l'animal.

a) En l'absence de mannane : cinétique d'élimination plasmatique de l'immunotoxine IT-T101, de l'anticorps T101 et de la chaîne A de ricine.

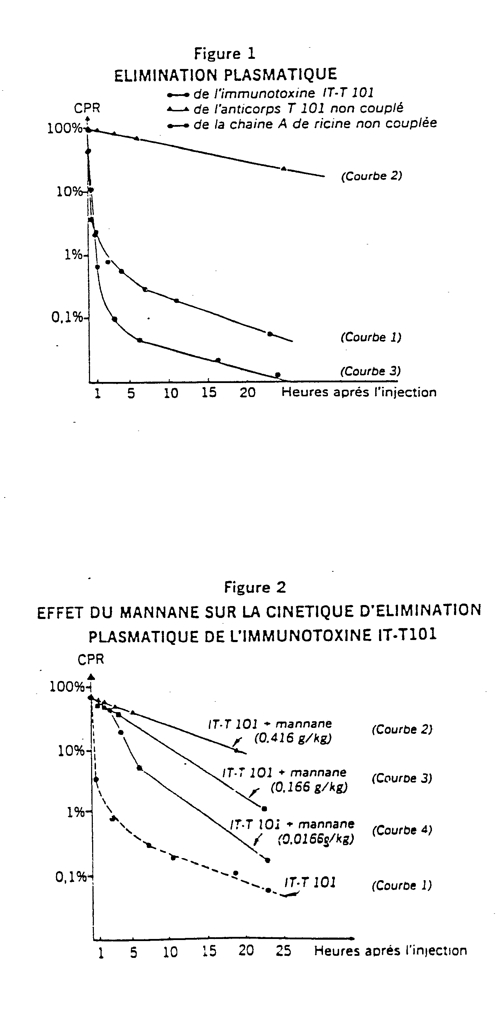

La figure 1 montre la courbe d'élimination plasmatique de l'IT-T101 qui présente deux phases (courbe 1). Dans la première phase, le produit disparaît rapidement (environ 97 % en 30 min) ; dans la deuxième phase, la décroissance est plus lente. La première phase d'élimination observée avec l'IT-T101 n'apparaît pas dans la cinétique d'élimination de l'anticorps T101 où seule une phase d'élimination, lente, est enregistrée (courbe 2). Par contre, la cinétique d'élimination plasmatique de la chaîne A non couplée est très comparable à celle de l'IT-T101 : 1 h après l'injection, il ne reste plus dans le plasma que 0,7 % de la dose administrée (courbe 3).

b) En présence de mannane : cinétique d'élimination plasmatique de l'immunotoxine IT-T101 et de la chaîne A de ricine.

L'administration du mannane a été effectuée selon le schéma suivant :

10 min avant l'injection de l'IT-T101 ou de la chaîne A, 20 % de la dose finale du polysaccharide sont injectés par voie intraveineuse. Au temps zéro, 40 % de la dose finale du polysaccharide sont injectés par voie intraveineuse en association avec l'IT-T101 ou la chaîne A (0,415 mg de chaîne A/kg). Puis, respectivement aux temps 1,5 h et 5 h, 20 % de la dose finale de polysaccharide sont injectés par voie intraveineuse.

La figure 2 montre la courbe d'élimination plasmatique en fonction du temps de l'IT-T101 injectée par voie intraveineuse en association avec le mannane à la dose totale de 0,416 g/kg (courbe 2). En présence de mannane, la première phase d'élimination — responsable de la disparition de la plus grande partie du produit — est pratiquement abolie, ce qui conduit à un accroissement majeur du taux d'immunotoxine plasmatique active. 15 h après l'injection, la concentration de l'IT-T101 est 100 fois supérieure, quand l'immunotoxine a été associée au mannane, à ce qu'elle est en l'absence de mannane (courbe 1).

Cet effet d'inhibition de l'élimination plasmatique de l'immunotoxine est dépendant de la dose de mannane. Des doses inférieures de mannane (166 mg/kg et 16,6 mg/kg) produisent des effets plus faibles (courbes 3 et 4).

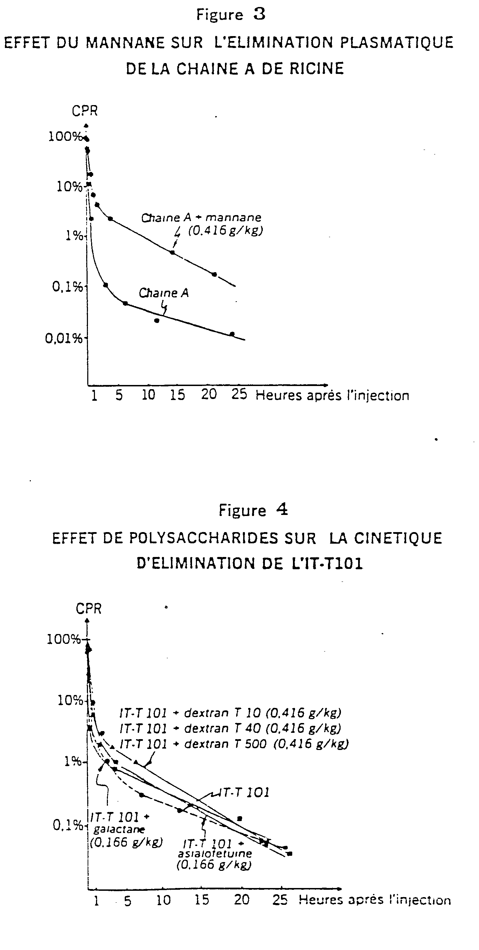

Les propriétés du mannane sont également observées avec la chaîne A non couplée comme le montre la figure 3. Ce point confirme que la rapide disparition de l'immunotoxine est bien imputable à la chaîne A constitutive, en particulier en raison de ses résidus mannosidiques terminaux.

On constate néanmoins que la première phase d'élimination de la chaîne A n'est pas totalement abolie, contrairement à ce qui se passe avec l'immunotoxine. Ceci confirme l'effet totalement surprenant observé lors de l'administration simultanée de mannane et d'immunotoxine.

Exemple 2

Afin de montrer la spécificité d'action éventuelle du mannane, la cinétique d'élimination plasmatique de l'immunotoxine IT-T101 a été mesurée en présence d'autres polysaccharides ne possédant pas de résidus mannose en position terminale. C'est le cas des dextrans T10, T40 ou T500 (polymères de glucose de poids moléculaires voisins de 10 000, 40 000 et 500 000, respectivement) administrés à la dose totale de 416 mg/kg, du galactane (polymère de galactose) administré à la dose totale de 166 mg/kg de l'asialofétuine (glycoprotéine fortement glycosylée à galactose terminal) administrée à la dose totale de 166 mg/kg.

Les courbes représentées sur la figure 4 montrent que ces polysaccharides sont pratiquement sans action sur la cinétique d'élimination plasmatique de l'immunotoxine IT-T101.

Exemple 3

Cet exemple démontre la captation hépatique de la chaîne A de ricine après injection intraveineuse et l'inhibition de cette captation par le mannane.

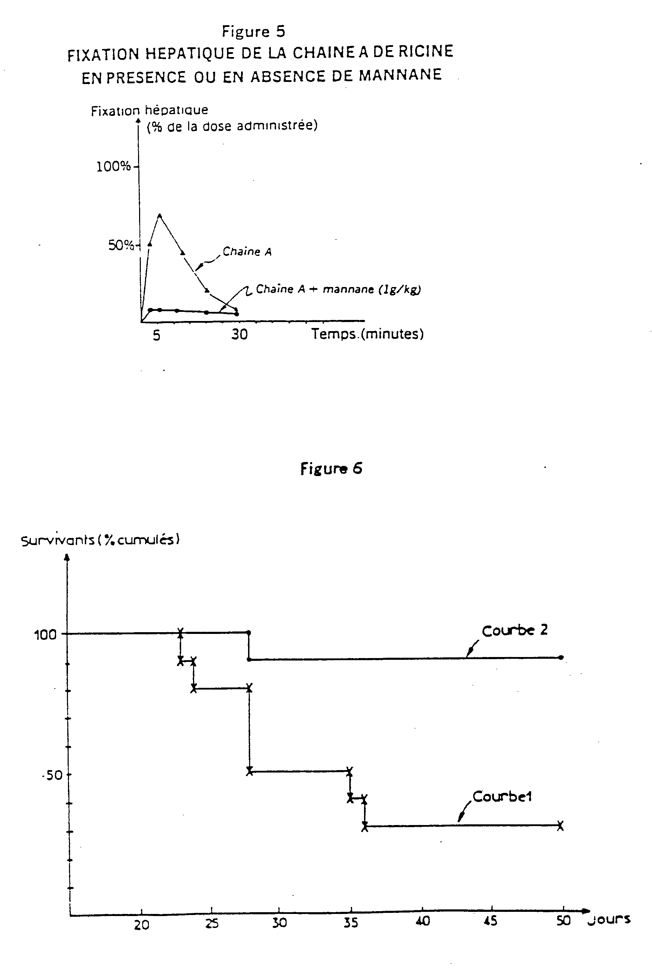

La chaîne A radiomarquée à l'iode 125 est injectée chez la souris Charles River France CD1 par voie intraveineuse en l'absence ou en présence de mannane à la dose de 1 g/kg. Aux différents temps de l'expérience, deux animaux sont anesthésiés. La cavité abdominale est ouverte, la veine cave sectionnée, le foie est lavé avec 10 ml d'eau physiologique par injection dans la veine porte. Le foie est prélevé dans sa totalité et la radioactivité est déterminée. Les résultats sont représentés en pourcentage du nombre de cpm fixés sur le foie par rapport au nombre de cpm totaux injectés (figure 5). En l'absence de mannane, la chaîne A de ricine est très précocement et efficacement captée par le foie comme l'indique le pic de radioactivité. Inversement, en présence de mannane, ce pic de radioactivité est pratiquement aboli. Ce résultat confirme que la chaîne A de ricine est piégée par le foie et que le maintien de l'immunotoxine comme de la chaîne A à des taux plasmatiques élevés en présence de mannane est bien dû à l'inhibition de cette capture hépatique.

Exemple 4

Cet exemple démontre l'absence d'effet antagoniste du mannane vis-à-vis de la cytotoxicité sélective de l'immunotoxine IT-T101 in vitro.

Dans ces expériences, la cytotoxicité a été évaluée par la mesure de l'incorporation de 14C-leucine par les cellules cibles (cellules CEM) après 24 h d'incubation à 37 °C en présence de concentrations connues de l'immunotoxine étudiée, ou de substances cytotoxiques de référence, en absence ou

5

présence de mannane à la concentration de 10 mg/ml. La technique employée est celle précédemment décrite (J. Biol. Chem. 1984, 259 (15), 9359).

Il a été préalablement vérifié que le mannane n'est pas cytotoxique pour les cellules aux concentrations utilisées. Les résultats de ces expériences sont présentés dans le tableau I. L'effet cytotoxique est mesuré par la valeur de la concentration molaire (CI50) exprimée en chaîne A qui entraîne une inhibition de l'incorporation du traceur de 50 %.

L'immunotoxine seule ou sous sa forme activée par le chlorure d'ammonium conserve pleinement son activité. De la même façon, la toxicité intrinsèque de la chaîne A n'est pas modifiée. Ainsi, en présence de mannane, les propriétés caractéristiques de cytotoxicité de l'immunotoxine ne sont pas altérées.

Tableau I

| Substances testées | CI50 exprimée en molarité de chaîne A | |
|---|---|---|
| | sans mannane | avec mannane (10mg/ml) |
| Immunotoxine IT-T101 plus $NH_4Cl$ | $3,0 \ 10^{-13}$ M | $2,8 \ 10^{-13}$ M |
| IT-T101 | $1,0 \ 10^{-9}$ M | $1,2 \ 10^{-9}$ M |
| Chaîne A | $7,0 \ 10^{-7}$ M | $7,0 \ 10^{-7}$ M |

## Exemple 5

Toxicité de l'immunotoxine IT-T101 injectée en association avec le mannane chez la souris.

Il était important de vérifier quel était l'impact toxicologique global sur l'animal entier de l'association immunotoxine plus mannane. Pour cela, nous avons déterminé la dose létale 50 % de l'immunotoxine antimélanome (IT-HM) administrée par voie intraveineuse chez la souris Charles River France CD1 en l'absence ou avec coadministration de 10 mg de mannane par souris par voie intraveineuse. La préparation et les propriétés cytotoxiques de ce conjugué antimélanome (IT-HM) ont été décrites dans la demande de brevet français n° 81/07596.

Les valeurs trouvées sont indiquées dans le tableau II.

Tableau II

| Produit testé | DL 50 |
|---|---|
| IT-HM seule | 460 microgrammes/souris |
| IT-HM + 10 mg mannane/souris | 115 microgrammes/souris |

Ces résultats montrent une légère augmentation de toxicité de l'immunotoxine lorsqu'elle est administrée en même temps que le mannane. Cet accroissement de toxicité par un facteur de seulement 4 ne constitue pas une limitation à l'emploi in vivo de mannane compte tenu des effets très importants vis-à-vis du maintien de la concentration plasmatique des immunotoxines in vivo, comme cela été démontré ci-dessus.

## Exemple 6

Cet exemple a pour but de mettre en évidence l'effet du mannane sur l'action d'une immunotoxine dans un essai « in vivo ».

L'expérimentation a été effectuée chez des souris BL 1.1 (Thy 1.2 négatives) [International Journal of Cancer 24, 168-177, (1979)]. L'immunotoxine utilisée est le conjugué associant par une liaison disulfure l'anticorps anti-Thy 1.2 (anticorps AT15E) et la chaîne A de la Ricine et préparé selon le procédé décrit dans les demandes de brevets français au nom de la demanderesse et citées précédemment.

Le protocole suivant a été utilisé.

Des groupes de 10 souris BL 1.1 reçoivent par injection intraveineuse le jour $0,5 \times 10^4$ cellules T2 (cellules Thy 1.2 positives de lymphome murin) et sont randomisés avant traitement.

Le traitement est effectué par voie intraveineuse le jour 1 :

1 lot reçoit 10 µg/souris du conjugué anticorps AT15E-chaîne A de ricine seul.

1 autre lot reçoit la même quantité du même conjugué mélangé avec 10 mg de mannane.

De plus 4 lots témoins reçoivent respectivement :

le milieu de culture RPMI (milieu utilisé pour la culture des cellules T2) ;

le mannane seul (10 mg/souris) ;

la chaîne A de ricine (10 µg) et le mannane (10 mg) ;

la chaîne A de ricine (10 µg), l'anticorps AT15E (30 µg) et le mannane (10 mg).

Les animaux sont observés pendant 50 jours et la mortalité est notée.

La figure 6 donne le pourcentage d'animaux survivants en fonction du temps écoulé après le traitement.

La courbe 1 concerne les animaux ayant reçu l'immunotoxine seule et la courbe 2 les animaux ayant reçu l'association immunotoxine plus mannane.

Ainsi qu'on peut le constater, l'association immunotoxine-mannane a donné 90 % de survivants 50 jours après le traitement alors que l'administration de l'immunotoxine seule n'a donné que 30 % d'animaux survivants après 50 jours.

De plus, sur les lots témoins (non représentés sur la figure 6), on a observé au jour 50 :

0 % de survivants pour les animaux traités par RPMI et par chaîne A plus mannane ;

10 % de survivants pour les animaux ayant reçu le mannane seul ;

20 % de survivants pour les animaux traités par chaîne A de ricine + anticorps + mannane.

Ces résultats montrent l'efficacité de l'association immunotoxine-mannane aussi bien vis-à-vis de l'immunotoxine utilisée seule que vis-à-vis des témoins.

On peut donc utiliser en tant que médicaments en thérapeutique humaine l'association constituée par une immunotoxine et du mannane. Cette association peut être utilisée pour le traitement des affections, cancéreuses ou non, dont les cellules cibles seraient reconnues par l'anticorps utilisé pour la préparation de l'immunotoxine.

Visant à éliminer la totalité des cellules cibles, le traitement devra être effectué avec une dose suffisante d'immunotoxine associée avec une quantité de mannane pouvant varier de 10 mg à 1 g/kg à chaque administration d'immunotoxine. Les modalités optimales d'administration des constituants de l'association ainsi que la durée du traitement devront être déterminées dans chaque cas en fonction du sujet et de la nature de l'affection à traiter.

Les nouveaux médicaments selon l'invention sont conditionnés pour être utilisés par voie injectable et, de préférence, par voie intraveineuse. De préférence, les constituants de l'association seront conservés séparés et pourront être mélangés en cas de besoin seulement au moment de l'emploi, dans la seringue ou le solvant de perfusion.

**Revendications** (pour les Etats contractants : BE, CH, DE, FR, GB, IT, LI, LU, NL, SE)

1. Médicaments conditionnés de façon à être utilisés par voie injectable, caractérisés en ce qu'ils comportent, en association, au moins une immunotoxine obtenue à partir de toxine ou sous-unité toxique ou fragment de sous-unité toxique naturelle ou semi-synthétique ou synthétique comportant des groupements polysaccharidiques contenant des résidus de mannose, en particulier en position terminale, quel que soit l'anticorps constitutif et quel que soit le type de liaison choisi pour relier l'anticorps à la toxine ou sous-unité toxique ou fragment toxique, et au moins un polymère glucidique, polyosidique ou polysaccharidique de poids moléculaire moyen supérieur à 1 000 comportant de 20 à 100 % de résidus mannose, quel que soit le type d'enchaînement reliant ces résidus mannose entre eux ou à d'autres sucres.

2. Médicaments selon la revendication 1, caractérisés en ce que l'immunotoxine est l'immunotoxine anti-T65 et le polymère contenant du mannose est un mannane.

3. Médicaments selon l'une quelconque des revendications 1 ou 2, caractérisés en ce qu'ils sont conditionnés de façon à être utilisés par voie intraveineuse.

4. Médicaments selon l'une quelconque des revendications 1 à 3, caractérisés en ce que l'immunotoxine et le polymère contenant du mannose sont conditionnés séparément.

**Revendications** (pour l'Etat contractant AT)

1. Utilisation en association d'au moins une immunotoxine obtenue à partir de toxine ou sous-unité toxique ou fragment de sous-unité toxique naturelle ou semi-synthétique ou synthétique comportant des groupements polysaccharidiques contenant des résidus de mannose, en particulier en position terminale, quel que soit l'anticorps constitutif et quel que soit le type de liaison choisi pour relier l'anticorps à la toxine ou sous-unité toxique ou fragment toxique, et d'au moins un polymère glucidique, polyosidique et polysaccharidique de poids moléculaire moyen supérieur à 1 000 comportant de 20 à 100 % de résidus mannose, quel que soit le type d'enchaînement reliant ces résidus mannose entre eux ou à d'autres sucres, pour la préparation de médicaments conditionnés de façon à être utilisés par voie injectable.

2. Utilisation selon la revendication 1, caractérisée en ce que l'immunotoxine est l'immunotoxine anti-T65 et le polymère contenant du mannose est un mannane.

3. Utilisation selon l'une des revendications 1 ou 2, caractérisée en ce que les médicaments sont conditionnés de façon à être utilisés par voie intraveineuse.

4. Utilisation selon l'une quelconque des revendications 1 à 3, caractérisée en ce que l'immunotoxine et le polymère contenant du mannose sont conditionnés séparément.

**Claims** (for the Contracting States : BE, CH, DE, FR, GB, IT, LI, LU, NL, SE)

1. Drugs packaged for administration by injection, characterized in that they comprise an association of at least one immunotoxin obtained from a natural, semi-synthetic or synthetic toxin, toxic sub-unit or fragment of toxic sub-unit comprising polysaccharide groups containing mannose residues, in particular in the terminal position, irrespective of the constituent antibody and irrespective of the type of bond chosen to join the antibody to the toxin, toxic sub-unit or toxic fragment, and at least a polyoside or polysaccharide carbohydrate polymer which has an average molecular weight greater than 1 000 comprising from 20 to 100 % of mannose residues, irrespective of the type of linkage joining these mannose residues to one another or to other sugars.

2. Drugs according to claim 1, characterized in that the immunotoxin is the immunotoxin anti-T65 and the mannose-containing polymer is a mannane.

3. Drugs according to any one of claims 1 or 2, characterized in that they are packaged for administration by intravenous injection.

4. Drugs according to any one of claims 1 to 3, characterized in that the immunotoxin and the mannose-containing polymer are packaged separately.

**Claims** (for the Contracting State AT)

1. Drugs packaged for administration by injection, characterized in that they comprise an association of at least one immunotoxin obtained from a natural, semi-synthetic or synthetic toxin, toxic sub-unit or fragment of toxic sub-unit comprising polysaccharide groups containing mannose residues, in particular in the terminal position, irrespective of the constituent antibody and irrespective of the type of bond chosen to join the antibody to the toxin, toxic sub-unit or toxic fragment, and at least a polyoside or polysaccharide carbohydrate polymer which has an average molecular weight greater than 1 000 comprising from 20 to 100 % of mannose residues, irrespective of the type of linkage joining these mannose residues to one another or to other sugars.

2. Drugs according to claim 1, characterized in that the immunotoxin is the immunotoxin anti-T65 and the mannose-containing polymer is a mannane.

3. Drugs according to any one of claims 1 or 2, characterized in that they are packaged for intravenous injection.

4. Drugs according to any one of claims 1 to 3, characterized in that the immunotoxin and the mannose-containing polymer are packaged separately.

**Patentansprüche** (für die Vertragsstaaten BE, CH, DE, FR, GB, IT, LI, LU, NL, SE)

1. Zur Verwendung auf Injektionsweg konditionierte Medikamente, dadurch gekennzeichnet, daß sie in Kombination mindestens ein Immunotoxin, erhalten aus Toxin oder der toxischen Untereinheit oder dem Fragment der natürlichen oder halbsynthetischen oder synthetischen toxischen Untereinheit mit insbesondere in terminaler Position Mannosereste enthaltenden Polysaccharidgruppen, unabhängig vom konstitutiven Antikörper und unabhängig von der zum Binden des Antikörpers an das Toxin oder die toxische Untereinheit oder das toxische Fragment gewählten Bindungstype, und mindestens ein Glyzid-, Polyosid- oder Polysaccharidpolymeres mit einem mittleren Molekulargewicht von mehr als 1 000 und mit 20 bis 100 % Mannoseresten, unabhängig von der diese Mannosereste untereinander oder mit anderen Zuckern verbindenden Art der Kettenbildung, umfassen.

2. Medikament nach Anspruch 1, dadurch gekennzeichnet, daß das Immunotoxin anti-T65-Immunotoxin und das mannosehältige Polymere ein Mannan ist.

3. Medikamente nach einem der Ansprüche 1 oder 2, dadurch gekennzeichnet, daß sie zur Verwendung auf intravenösem Weg Konditioniert sind.

4. Medikamente nach einem der Ansprüche 1 bis 3, dadurch gekennzeichnet, daß das Immunotoxin und das mannosehältige Polymere getrennt konditioniert sind.

**Patentansprüche** (für den Vertragsstaat AT)

1. Verwendung in Kombination mindestens eines Immunotoxins, erhalten aus Toxin oder der toxischen Untereinheit oder dem Fragment der natürlichen oder halbsynthetischen oder synthetischen toxischen Untereinheit mit insbesondere in terminaler Position Mannosereste enthaltenden Polysaccharidgruppen, unabhängig vom konstitutiven Antikörper und unabhängig von der zum Binden des Antikörpers an das Toxin oder die toxische Untereinheit oder das toxische Fragment gewählten Bindungstype, und mindestens eines Glyzid-, Polyosid- und Polysaccharidpolymeren mit einem mittleren Molekulargewicht von mehr als 1 000 und mit 20 bis 100 % Mannoseresten, unabhängig von der diese Mannosereste untereinander oder mit anderen Zuckern verbindenden Art der Kettenbildung, zur Herstellung von zur Verwendung auf Injektionsweg konditionierten Medikamenten.

2. Verwendung nach Anspruch 1, dadurch gekennzeichnet, daß das Immunotoxin anti-T65-Immunotoxin und das mannosehältige Polymere ein Mannan ist.

3. Verwendung nach einem der Ansprüche 1 oder 2, dadurch gekennzeichnet, daß die Medikamente zur Verwendung auf intravenösem Weg konditioniert sind.

4. Verwendung nach einem der Ansprüche 1 bis 3, dadurch gekennzeichnet, daß das Immunotoxin und das mannosehältige Polymere getrennt konditioniert sind.

Figure 1
ELIMINATION PLASMATIQUE

— de l'immunotoxine IT-T 101
— de l'anticorps T 101 non couplé
— de la chaine A de ricine non couplée

(Courbe 2)

(Courbe 1)

(Courbe 3)

CPR
100%
10%
1%
0,1%

1  5  10  15  20  Heures après l'injection

Figure 2

EFFET DU MANNANE SUR LA CINETIQUE D'ELIMINATION
PLASMATIQUE DE L'IMMUNOTOXINE IT-T101

CPR
100%
10%
1%
0,1%

IT-T 101 + mannane
(0.416 g/kg)          (Courbe 2)

IT-T 101 + mannane
(0.166 g/kg)          (Courbe 3)

IT-T 101 + mannane
(0.0166g/kg)          (Courbe 4)

IT-T 101              (Courbe 1)

1  5  10  15  20  25  Heures après l'injection

1

Figure **3**

EFFET DU MANNANE SUR L'ELIMINATION PLASMATIQUE

DE LA CHAINE A DE RICINE

Figure **4**

EFFET DE POLYSACCHARIDES SUR LA CINETIQUE

D'ELIMINATION DE L'IT-T101

2

Figure 5

## FIXATION HEPATIQUE DE LA CHAINE A DE RICINE
## EN PRESENCE OU EN ABSENCE DE MANNANE

Figure 6